# EUROPÄISCHE PATENTSCHRIFT

(11) **EP 1 931 625 B1**
(45) Veröffentlichungstag und Bekanntmachung des Hinweises auf die Patenterteilung: **11.11.2015**
(21) Anmeldenummer: 06793700.3
(22) Anmeldetag: 21.09.2006
(51) Int. Cl.: C07C 263/20, C07C 265/14

(54) **VERFAHREN ZUR AUFREINIGUNG VON ISOCYANATHALTIGEN RÜCKSTÄNDEN**
METHOD FOR PURIFYING RESIDUES CONTAINING ISOCYANATES
PROCEDE POUR PURIFIER DES RESIDUS CONTENANT DES ISOCYANATES

(30) Priorität: 29.09.2005 DE 102005046816; 25.10.2005 DE 102005051399
(43) Veröffentlichungstag der Anmeldung: 18.06.2008
(73) Patentinhaber: BASF SE, 67056 Ludwigshafen (DE)
(72) Erfinder: WÖLFERT, Andreas, 74906 Bad Rappenau (DE); KNÖSCHE, Carsten, 67150 Niederkirchen (DE); KLÖTZER, Matthias, 01945 Kroppen (DE); ASCHERL, Hermann, 67246 Dirmstein (DE); STROEFER, Eckhard, 68163 Mannheim (DE); BLANKERTZ, Heinrich-Josef, 67147 Forst (DE); SCHÖNHERR, Michael, 67227 Frankenthal (DE); KARCHES, Martin, 67434 Neustadt (DE); BENZ, Christian, 67549 Worms (DE)
(86) Internationale Anmeldenummer: PCT/EP2006/066577
(87) Internationale Veröffentlichungsnummer: WO 2007/036479

(56) Entgegenhaltungen:
- EP-A1- 0 626 368
- US-A- 3 405 040
- US-A- 5 962 728
- US-A1- 2005 159 495

## Beschreibung

Die vorliegende Erfindung umfaßt ein Verfahren zur Aufreinigung von isocyanathaltigen Rückständen.

Bei der Herstellung von Diisocyanaten fällt technisch ein überwiegend polymerer Rückstand an, der noch signifikante Anteile an verwertbarem Produkt enthalten kann, das zur Verbesserung der Ausbeute des Verfahrens abgetrennt werden soll. Zudem sind die monomeren Diisocyanate zumeist toxisch, so daß ein Gehalt an monomerem Diisocyanat im Rückstand diesen in der Handhabung beispielsweise für die Entsorgung gefährlich machen kann.

Je nach Natur des Isocyanats ist der Rückstand fest oder bildet eine zähviskose, klebrige und demzufolge schwer förderbare Masse.

Verfahren zur Abtrennung von monomerem Diisocyanat aus Rückständen aus dem Phosgenverfahren sind prinzipiell bekannt.

US 4,216,063 beschreibt ein Verfahren zur Abtrennung von monomerem Diisocyanat aus Toluoldiisocyanat (TDI), dessen Herstellverfahren nicht weiter ausgeführt wird, mit Hilfe eines bewegten und selbstreinigenden Verdampferapparates. TDI bildet unter den offenbarten Reaktionsbedingungen (Temperatur 70 bis 250 °C, Druck 1 - 50 mm Hg) einen porösen, spröden Feststoff, der durch die Mahlbewegungen der inneren Organe des Verdampferapparates in ein rieselfähiges Gut überführt wird Mithin ist diese Lösung für solche Diisocyanate, die nicht versprödende Rückstände bilden, nicht anwendbar.

EP 626 368 A1 beschreibt ein Verfahren zur Abtrennung von monomerem Diisocyanat aus Produktionsrückständen von Toluoldiisocyanat (TDI), dessen Herstellung nicht weiter ausgeführt wird, unter Zugabe hochsiedender Kohlenwasserstoffe, wie beispielsweise Bitumen, zur Vermeidung von Staubbildung. Man erhält dabei auch hier in einem Schaufeltrockner rieselfähiges Produkt, das über eine Förderschnecke oder Zellradschleuse ausgetragen werden kann. Nachteilig an dem Verfahren ist der Aufwand für die Bereitstellung und Handhabung eines zusätzlichen Stoffes, nämlich des hochsiedenen Kohlenwasserstoffs in der Anlage. Im Vergleich zu einem Verfahren, das ohne Hilfsstoffe auskommt, ergibt sich weiterhin ein erhöhter Anfall an zu deponierenden oder zu verbrennenden Abfällen.

US 5,962,728 schlägt für flüssig-viskose Rückstände-bildende Diisocyanate, wie z.B. HDI, IPDI oder H12MDI, die aus Phosgenierungsprozessen stammen, vor, einen Verdampfertrockner einzusetzen, der unterteilt ist in eine Heizzone (Temperatur 250 - 280 °C und Druck 1 - 5 mm Hg) und eine Kühlzone (Temperatur 100 - 120 °C und Druck 1 - 50 mm Hg). Durch die Abkühlung in der nachgeschalteten Kühlzone erstarrt das Produkt im Apparat zu einem spröden Feststoff, der nach Feinmahlen durch die inneren Organe des Apparates als rieselfähiger Feststoff ausgetragen wird.

Nachteilig daran ist, daß durch die Aufteilung in eine Heiz- und eine Kühlzone innerhalb des Apparats aufgrund der unterschiedlichen Ausdehnung Anfahr- und insbesondere Dichtigkeitsprobleme entstehen. Zudem wird durch den ständigen Wärmefluß aus der Heiz- in die Kühlzone der Energieverbrauch erhöht.

WO 2004/56759 beschreibt die Rückgewinnung von Diisocyanat aus Rückstandsströmen mittels Schaufeltrockner. Die Diisocyanate können dabei bevorzugt durch Phosgenierung erhalten werden, denkbar wäre jedoch auch die Herstellung über ein Harnstoffverfahren. Als Diisocyanate werden "alle gängigen (cyclo)aliphatischen und aromatischen Isocyanate" beschrieben.

Nachteilig an allen vorgenannten Schriften ist, daß diese sich auf Diisocyanatherstellverfahren beziehen, die aus einer Phosgenierung stammen. Die Rückstände aus diesen Verfahren weisen einen deutlichen Chlorgehalt auf, der zum einen Korrosion verursacht und deswegen höhere Anforderungen an den Werkstoff stellt und zum anderen ein völlig anderes Nebenproduktspektrum aufweist als Rückstände, die aus einem phosgenfreien Prozeß stammen.

Ferner ist nachteilig, daß die offenbarten Verfahren die Diisocyanate nicht nach ihren Stoffeigenschaften differenzieren, die die Diisocyanate besser oder weniger geeignet für die Rückgewinnung machen.

Soweit Feststoffe gebildet werden so müssen diese spröde sein und soweit flüssigviskose Rückstände gebildet werden müssen diese in einen spröden Feststoff umgewandelt werden.

Aufgabe der vorliegenden Erfindung war es also, ein Verfahren zur Abtrennung von Monomeren aus Diisocyanatrückständen zur Verfügung zu stellen, mit dem die Rückstände verfahrenstechnisch einfach behandelt werden können.

Die Aufgabe wurde gelöst durch ein Verfahren zur Abtrennung von Monomeren aus einem diisocyanathaltigen Rückstand, in mindestens einem Apparat, in dem der Rückstand während der gesamten Verweilzeit im Apparat bei einer Temperatur zwischen 210 und 330 °C und einem Druck unter 300 hPa eine hochviskose Flüssigkeit und/oder einen nicht-versprödenden Feststoff bildet und durch eine Zwangsförderung für nichtfeste Medien aus diesem Apparat ausgetragen wird.

Es stellt einen Vorteil der vorliegenden Erfindung dar, daß mit den ausgewählten Apparaten der Austrag des monomerabgereicherten Rückstandes aus dem Apparat vereinfacht möglich ist und ferner nicht erforderlich ist, daß der Rückstand einen spröden Feststoff bilden muß.

Diisocyanate, deren Rückstände in dem erfindungsgemäßen Verfahren behandelt werden können, sind (cyclo)aliphatische Diisocyanate besonders bevorzugt Diisocyanate mit 4 bis 20 C-Atomen. Beispiele für übliche Diisocyanate sind aliphatische Diisocyanate wie Tetramethylendiisocyanat, Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, Derivate des Lysindiisocyanates, Tetramethylxylylendiisocyanat, Trimethylhexandiisocyanat oder Tetramethylhexandiisocyanat, sowie cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5- trimethyl-5-(isocyanatomethyl)cyclohexan (Isophorondiisocyanat), 1,3- oder 1,4-Bis(isocyanatomethyl)cyclohexan, 2,4-, oder 2,6-Diisocyanato-1-methylcyclohexan sowie 3 (bzw. 4), 8 (bzw. 9)-Bis(isocyanatomethyl)-tricyclo[5.2.1.0^{2.6}]decan-Isomerengemische.

Unter diesen bevorzugt sind 1,6-Diisocyanatohexan, 4,4'- oder 2,4'-Di(isocyanatocyclohexyl)methan und 1-Isocyanato-3,3,5- trimethyl-5-(isocyanatomethyl)cyclohexan, besonders bevorzugt ist 1,6-Diisocyanatohexan.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

(Cyclo)aliphatische Isocyanate steht im Rahmen dieser Anmeldung kurz für cycloaliphatische und/oder aliphatische Isocyanate.

Als monomerhaltige Rückstände in das erfindungsgemäße Verfahren einsetzbar sind die Rückstände solcher (cyclo)aliphatischer Diisocyanate, deren monomerhaltigen Rückstände während der gesamten Verweilzeit innerhalb des Apparates unter den dort herrschenden Bedingungen eine hochviskose Flüssigkeit und/oder einen nicht versprödenden Feststoff bilden.

Unter "hochviskos" wird hier eine Viskosität von mehr als 1000 mPas gemäß DIN EN ISO 3219 unter den im Apparat herrschenden Bedingungen verstanden.

Unter "pastös" wird hier ein flüssiges oder hochviskoses Medium verstanden, daß Feststoffanteile enthält aber rheologisch unter den im Apparat herrschenden Bedingungen fließfähig aber unter dem alleinigen Einfluß der Erdbeschleunigung nicht fließfähig ist.

Unter "spröde" wird hier die Eigenschaft von Stoffen verstanden, unter Belastung zu brechen oder zu reißen. Die Sprödigkeit tritt im allgemeinen unterhalb einer Übergangstemperatur T_{Ü} auf, oberhalb der sich der Stoff zäh verhält. Spröde sind solche Stoffe, die im Spannungs-Dehnungs-Diagramm, das man erhält, wenn man eine Probe mit einer Zugkraft F belastet und die verursachte Längenänderung ΔL darüber aufträgt, eine steile Hookesche Gerade aufweisen, die den Proportionalbereich von Dehnung und Spannung kennzeichnet, wobei die Hookesche Gerade mit dem Bruch endet.

Allgemein können Diisocyanate industriell im wesentlichen entweder durch Phosgenierung von Diaminen erhalten werden, z.B. nach Verfahren basierend auf den in DE-PS 20 05 309 und DE-OS 2 404 773 beschriebenen Verfahren, oder durch phosgenfreie Verfahren (Spaltung von Biurethanen), wie z.B. beschrieben in der EP-B 126 299 (US-A-4 596 678), EP-B 126 300 (US-A-4 596 679), EP-A 355 443 (US-A-5 087 739), EP 566 925 A2 sowie in der EP-A 568 782 hergestellt werden.

Die Herstellung nach einem Phosgenverfahren erfolgt durch Phosgenierung der entsprechenden Diamine und thermische Spaltung der intermediär gebildeten Dicarbaminsäurechloride. Isocyanate, die aus einem Phosgenierungsprozeß stammen, weisen in der Regel einen Gesamtchlorgehalt von 100-700 mg/kg auf. Als hochsiedende Verunreinigungen treten vor allem chlorhaltige Nebenkomponenten auf.

Die Herstellung nach phosgenfreien Verfahren, auch Harnstoffverfahren genannt, erfolgt vorzugsweise durch thermische Spaltung der entsprechenden Carbamate. Diese Spaltung wird bei Temperaturen von 150 bis 300 °C, meist unter Verwendung von Katalysatoren, durchgeführt. Die bei der Spaltung entstehenden Diisocyanate und Alkohole werden, zumeist durch Destillation, aus dem Reaktionsgemisch entfernt und gereinigt.

Nach letzterem Verfahren erhaltene Diisocyanat weisen einen Gesamtchlorgehalt von weniger als 80 mg/kg auf, bevorzugt weniger als 60, besonders bevorzugt weniger als 40, ganz besonders bevorzugt weniger als 20, insbesondere weniger als 10 mg/kg und speziell von 0 mg/kg.

Es ist prinzipiell möglich, in das erfindungsgemäße Verfahren diisocyanathaltige Rückstande einzusetzen, die aus einem Phosgenverfahren oder aus einem phosgenfreien Verfahren stammen. In einer bevorzugten Ausführungsform der vorliegendenen Erfindung werden diisocyanathaltige Rückstande eingesetzt, die aus einem phosgenfreien Verfahren stammen.

Werden diisocyanathaltige Rückstande eingesetzt, die aus einem Phosgenverfahren stammen, so kann es sinnvoll sein, den Apparat zumindest teilweise an den thermisch belasteten Stellen, an denen der Apparat dem chlorhaltigen Rückstand ausgesetzt ist, aus nichtrostendem Stahl zu fertigen. Die DIN-EN 10088-1 in der Fassung vom August 1995 definiert nichtrostende Stähle als solche, die mindestens 10,5 % Chrom und höchstens 1,2 % Kohlenstoff enthalten. Bevorzugt werden dann in dem erfindungsgemäßen Verfahren Apparate eingesetzt, die zumindest teilweise aus austenitischen und/oder austenitisch-ferritischen Stählen gefertigt sind.

Austenitische Stähle sind solche mit austenitischem Gittertyp (γ-Phase) bei 20 °C. Bevorzugt weisen sie einen Cr-Gehalt von 16 bis 28 % und einen Ni-Gehalt von 3,5 bis 32 % auf, sowie gegebenenfalls Anteile von S (bis zu 0,35%), P (bis zu 0,045%), Mo (bis zu 7%), Si (bis zu 4,5%), Cu (bis zu 4%), N (bis zu 0,25%) und/oder Mn (bis zu 10,5%), sowie eventuell Ti (bis zu 0,7%) und/oder Nb (bis zu 1%). Der Kohlenstoffgehalt beträgt in der Regel unter 0,15%. Unter diesen sind die hochlegierten austenitischen 18/8 Chrom-Nickelstähle besonders bevorzugt.

Austenitisch-ferritische Stähle weisen ein Zweiphasengefüge aus Ferrit und Austenit mit einem Ferrit-Anteil von ca. 60% auf. Der Cr-Anteil beträgt meist 19 - 28 %, Ni 3,5 - 8%, Mo bis zu 4,5% und gegebenenfalls Anteile von Mn (bis zu 2%), Cu (bis zu 2,5%), N (bis zu 0,35%), W (bis zu 1 %), S (bis zu 0,015%), Si (bis zu 1 %)und/oder P (bis zu 0,035%). Der Kohlenstoffgehalt beträgt in der Regel unter 0,05%.

Ganz besonders bevorzugte Werkstoffe sind die in der DIN-EN 10088-1 aufgeführten austenitischen und austenitisch-ferritischen Werkstoffe und insbesondere bevorzugt sind die Werkstoffe 1.4539 (Falk-Stahl), 1.4541, 1.4571 und 1.4462, sowie ferner auch Hastelloy A und C und Zirkon. Die genannten Werkstoffe gemäß DIN-EN 10088 entsprechen näherungsweise folgenden Werkstoffen gemäß AISI (American Iron and Steel institute), UNS (Unified Numbering System), SS (Swedish Standard), AFNOR (Association Francaise de Normalisation), BS (British Standard) und JIS (Japanese Industrial Standards):
1.4462 (X 2 CrNiMoN 22 5 3): UNS: S 31803, SS: 2377, AFNOR: Z 5 CNDU 21.08, JIS: SUS 329 J3L
1.4539 (X 1 NiCrMoCuN 25 20 5): UNS: N 08904, SS: 2562, AFNOR: Z 1 NCDU 25.20 1.4541 (X 6 CrNiTi 18 10): AISI: 321, UNS: S 32100, SS: 2337, AFNOR: Z 6 CNT 18.10, BS: 321 S 31, JIS: SUS 321
1.4571 (X 6 CrNiMoTi 17 12 2): AISI: 316 Ti, UNS: S 31635, SS: 2350, AFNOR: Z 6 CNDT 17.12, BS: 320 S 31, JIS: SUS 316 Ti

Unter den angeführten Werkstoffen sind solche mit erhöhten Chrom-, Kupfer-, Molybdän- und/oder Nickelanteilen vorteilhaft.

In einer bevorzugten Ausführungsform der vorliegenden Erfindung stammt der diisocyanathaltige Rückstand aus einem phosgenfreien Prozeß.

Die einfache Übertragung von Rückstandsaufarbeitungen aus Phosgenverfahren ist nicht möglich, da Produktströme in phosgenfrei hergestellten Verfahren ein gänzlich anderes Nebenproduktspektrum aufweisen und mithin auch andere Trennprobleme mit sich bringen.

Trotz des veränderten Nebenproduktspektrums ist es mit dem erfindungsgemäßen Verfahren nicht nur möglich, in dem Rückstand enthaltenes monomeres Diisocyanat zurückzugewinnen, sondern zusätzlich dazu auch darin enthaltenes Mono- und/oder Diurethan sowie andere verwertbare Produkte zurückzuspalten, so daß die effektive Ausbeute an monomerem Diisocyanat mit dem erfindungsgemäßen Verfahren bei den Rückständen aus einem phosgenfreien Verfahren höher sind, als bei Rückständen aus einem Phosgenverfahren.

Eine typische phosgenfreie Herstellung ist beispielsweise beschrieben in EP 566925 A2, Spalte 6, Z. 31 bis Spalte 8, 58.

Zur Herstellung (Urethanisierung) werden allgemein die zu den Diisocyanaten korrespondierenden Diamine mit Harnstoff und einem Alkohol gegebenenfalls in Gegenwart von Dialkylcarbonaten und/oder Carbamidsäureestern und gegebenenfalls in Gegenwart von Katalysatoren bei Temperaturen von 150 bis 300 °C unter einem Druck von 0,1 bis 60 bar innerhalb von 0,5 bis 50 Stunden zur Reaktion gebracht. Als Alkohole werden bevorzugt Methanol, Ethanol oder n-Butanol eingesetzt. Bei dieser Reaktion werden idealerweise die Diurethane gebildet, die in einer Folgestufe zu den Diisocyanaten gespalten werden.

Die erhaltene Diurethane enthaltende Reaktionsmischung wird dann in einer geeigneten Vorrichtung in Gegenwart oder bevorzugt in Abwesenheit von Lösungsmittel in flüssiger Phase bevorzugt in Gegenwart von Katalysatoren bei Temperaturen von 200 bis 300 °C und unter vermindertem Druck von 0,1 bis 200 mbar kontinuierlich thermisch gespalten. Der Umsatz von von Diurethan zu Diisocyanat in der Vorrichtung zur thermischen Spaltung kann in Abhängigkeit vom verwendeten Urethan weitgehend frei gewählt werden und liegt zweckmäßigerweise in einem Bereich von 10 bis 95 Gew.% der zugeführten Urethanmenge.

Der ungespaltene Anteil der Reaktionsmischung, der nicht umgesetzte Diurethane, Oligoharnstoff-diurethane, hochsiedende Oligomere und andere wiederverwertbare und unverwertbare Nebenprodukte enthält, wird abgetrennt, kontinuierlich aus der Spaltvorrichtung ausgeschleust und direkt oder gegebenenfalls nach Umsetzung mit Alkohol in das erfindungsgemäße Verfahren eingesetzt Eine solche Umsetzung (Reurethanisierung) kann beispielsweise erfolgen, wie in DE 10338511 A1, Absatz 0033 beschrieben, auf die hiermit voll inhaltlich Bezug genommen sei.

Die bei der thermischen Spaltung gebildeten Spaltprodukte, die sich vor allem aus Alkohol, Diisocyanat und partiell gespaltenen Urethanen zusammensetzen, werden danach vorteilhafterweise mit Hilfe einer oder mehrerer Destillationskolonnen, vorzugsweise durch Rektifikation bei Temperaturen von 100 bis 220°C und einem Druck von 1 bis 200 mbar in Alkohol und eine rohe Diisocyanatmischung mit einem Diisocyanatgehalt von 85 bis 99 Gew.% getrennt (Destillative Reinigung). Die Destillationskolonnen weisen in der Regel jeweils 1 bis 50 theoretische Trennstufen auf und sind von an sich bekannter Bauart. Die bei der destillativen Trennung anfallenden höhersiedenden Nebenprodukte und insbesondere die ungespaltenen und partiell gespaltenen Polyurethane können ebenfalls in das erfindungsgemäße Verfahren eingesetzt werden. Die nach phosgenfreien Verfahren erhaltenen Rückstände weisen als Nebenprodukte keine Chlorverbindungen auf und besitzen daher herstellungsbedingt ein grundsätzlich anderes Nebenproduktspektrum. Insbesondere sind als Nebenprodukte Mono- und/oder Diurethane, sowie Allophanate, Biurete und/oder Uretdione im Rückstand enthalten, die durch thermische Rückspaltung zu Diisocyanaten in den Apparaten erfindungsgemäß zumindest teilweise wiedergewonnen werden können.

Ein zusätzlicher Vorteil des erfindungsgemäßen Verfahrens für Rückstände, erhalten aus einem phosgenfreien Verfahren, ist, daß in der Regel keine besonderen Anforderung an korrosionsfeste Werkstoffe zu stellen sind, so daß an die Werkstoffe, aus denen der Apparat gefertigt ist, nicht, wie bei Rückständen, die aus einem Phosgenprozeß stammen, nichtrostende Stähle verwendet werden sollten, sondern der Apparat zusätzlich zu nichtrostenden Werkstoffen und bevorzugt vollständig aus Normalstahl gefertigt sein kann.

Als Rückstand, der dem Apparat zugeführt wird, kann beispielsweise ein hochsiederhaltiger Destillations- oder Rektifikationsrückstand des Reaktionsaustrages der Diisocyanatbildung eingesetzt werden. Destillationsvorrichtungen werden in der Regel bei 1 bis 80 mbar betrieben und einer Sumpftemperatur von 100 bis 240 °C. Die Rückstände werden in der Regel als Sumpfabzug erhalten.

Die Rückstände aus phosgenfreien Verfahren enthalten üblicherweise neben monomerem Diisocyanat auch dessen Polyisocyanate, insbesondere uretdion-, biuret- und/oder isocyanuratgruppenhaltige Polyisocyanat. Es ist eine Besonderheit von Diisocyanat, das über ein phosgenfreies Verfahren hergestellt worden ist, daß der Rückstand auch Mono- oder Diurethane, Allophanate und/oder Harnstoffe enthält, die unter den erfindungsgemäßen Abtrennungsbedingungen zumindest teilweise Wertprodukt, insbesondere zu monomerem Diisocyanat rückgespalten werden können.

Der Gehalt an Monomeren im Rückstand hängt von der vorgeschalteten Abtrennung des Diisocyanats ab. Der Gehalt an Monomeren kann bis zu 90 Gew% betragen, bevorzugt bis zu 80, besonders bevorzugt bis zu 70 und ganz besonders bevorzugt bis zu 60 Gew%.

Der monomerhaltige Rückstand, der in den Apparat zur Monomerwiedergewinnung geführt wird, wird in dieser Schrift vereinfacht als "Rückstand" bezeichnet, wohingegen zur Unterscheidung der den Apparat verlassenden Austrag als "monomerabgereicherter Rückstand" bezeichnet wird.

Mit dem Begriff "Apparat" wird hier ein verfahrenstechnischer Apparat bestimmt, in dem Monomer vom Rückstand abgetrennt und anschließend abgeführt werden kann.

Das Monomer wird dabei vom Rückstand durch einen destillativen und/oder Strippprozeß abgetrennt, der Rückstand wird im Apparat durchmischt.

Die Temperatur des Rückstandes innerhalb des Apparates beträgt erfindungsgemäß zwischen 210 und 330 °C, bevorzugt zwischen 225 und 305 °C, besonders bevorzugt von 235 bis 290 °C, ganz besonders bevorzugt von 245 bis 275 °C und insbesondere von 255 bis 265 °C. Durch das Austreiben niedrigsiedender Inhaltsstoffe aus dem Rückstand innerhalb des Apparates wird die Temperatur des Rückstandes innerhalb des Apparates in der Regel ansteigen.

Der Rückstand kann bei bestimmten Zusammensetzungen zum Aufschäumen neigen, dabei setzt die Schaumbildung bei einer bestimmten unteren Temperaturgrenze ein und verringert sich wieder bei einer oberen Temperaturgrenze. Ist dies der Fall, so stellt es eine vorteilhafte erfindungsgemäße Ausführungsform dar, den Rückstand mit einer Temperatur in den Apparat zu führen, die oberhalb der Temperaturgrenze liegt, bei der sich die Schaumbildung wieder verringert.

Die Temperatur, mit der der Rückstand in den Apparat geleitet wird beträgt bevorzugt mehr als 120 °C und weniger als 240 °C, besonders bevorzugt mehr als 150 °C und weniger als 220 °C, ganz besonders bevorzugt mehr als 180 °C und weniger als 210 °C.

Der Druck innerhalb des Apparates beträgt erfindungsgemäß unter 300 hPa, bevorzugt unter 200 hPa und besonders bevorzugt unter 100 hPa.

Die obere Verweilzeitgrenze innerhalb des Apparates beträgt in der Regel bis zu 5 Stunden, bevorzugt bis zu 3,5 Stunden, besonders bevorzugt bis zu 2,5, ganz besonders bevorzugt bis zu 2 und im speziellen bis zu 1,5 Stunden.

Die untere Verweilzeitgrenze innerhalb des Apparates beträgt erfindungsgemäß mindestens 5 Minuten, bevorzugt mindestens 10 Minuten, besonders bevorzugt mindestens 15 und ganz besonders bevorzugt mindestens 20 Minuten.

Der Apparat ist erfindungsgemäß zwangsaustragend. Dabei wird unter dem Begriff "zwangsaustragend" auch verstanden, wenn durch konstruktive Maßnahmen die Gutaustragsgeschwindigkeit über die natürliche, sich unter dem Einfluß des Erdschwerefeldes einstellende, Austragsgeschwindigkeit erhöht wird.

Zusätzlich ist es sinnvoll, den Apparat zwangsfördernd und/oder mit einem Fördergradienten zur Förderung des Rückstandes innerhalb des Apparates auszugestalten.

Unter dem Begriff "zwangsfördernd" wird im Rahmen der vorliegenden Erfindung verstanden, daß der in den Apparat eingetragene Rückstand durch Einbringen von mechanischer Energie durch den Apparat bewegt wird.

Die Förderung durch den Apparat erfolgt rückvermischungsarm oder rückvermischungsfrei. Diese Fördercharakteristik ist durch eine Bodensteinzahl von mindestens 3, bevorzugt mindestens 5, besonders bevorzugt mindestens 7 gekennzeichnet.

Bevorzugt wird zur Einengung der Verweilzeitverteilung im Schaufeltrockner der produktführende Innenraum mit blendenartigen Scheiben in verschiedene Segmente getrennt. Besonders bevorzugt werden mindestens zwei Scheiben eingesetzt.

In einer bevorzugten Ausführungsform erfolgt der axiale Transport durch den Apparat durch Anordnung von Förder-, Knet- und/oder Mischelementen, beispielsweise Scheibenelementen, Wellen, Schnecken, Blättern, Wischern oder Rotoren.

Im Apparat ist in der Regel ein mechanischer Energieeintrag von 5 W/kg oder mehr ausreichend, bevorzugt 10 oder mehr W/kg, besonders bevorzugt 20 oder mehr, ganz besonders bevorzugt 40 oder mehr, insbesondere 80 oder mehr und speziell 100 W/kg oder mehr. In der Regel bringt ein Energieeintrag von mehr als 200 W/kg keine Vorteile. Der angegebene spezifische Leistungseintrag ist hier als eingetragene Leistung pro Menge an Rückstand im Apparat.

Bevorzugt ist, dass der Schaufeltrockner eine Zwangsförderung in axialer Richtung aufweist. Die Zwangsförderung wird beispielsweise durch eine Schrägstellung der Oberflächen der Förderelemente erreicht.

Weiterhin ist vorteilhaft, dass der Schaufeltrockner eine Zwangsreinigung der inneren, produktberührten Oberflächen von mindestens 50 %, bevorzugt mindestens 60 %, ganz besonders bevorzugt von mindestens 70 % und im speziellen mindestens 80 % dieser inneren, produktberührten Oberflächen aufweist. Die Zwangsreinigung wird die Nähe der Förderelemente zur Aussenwandung bzw. durch die Nähe von Reinigungshaken zu den Förderelementen gewährleistet.

Zudem werden durch das erfindungsgemäße Verfahren nicht nur monomere Diisocyanate wiedergewonnen, sondern auch deren Folgeprodukte, soweit sie abtrennbar und/oder rückspaltbar sind.

Der Restgehalt an Monomeren im monomerabgereicherten Rückstand nach der Abtrennung beträgt in der Regel weniger als 20, bevorzugt weniger als 15, besonders bevorzugt weniger als 10, ganz besonders bevorzugt weniger als 5 und insbesondere weniger als 1 Gew%.

Es kann erfindungsgemäß sinnvoll sein, das Monomer nicht so vollständig abzutrennen, wie es apparativ möglich wäre, wenn dadurch der Austrag fest würde. Stattdessen macht es in diesem Fall erfindungsgemäß Sinn, einen Teil des Monomeren im aus dem Apparat ausgetragenen monomerabgereicherten Rückstand zu belassen, wenn dieser dadurch nicht fest wird. Dies ist insbesondere dann sinnvoll, wenn die Einsparung durch den vereinfachten Apparat den Verlust an Monomeren überkompensiert.

In diesem Fall beträgt der Restgehalt an Monomeren nach der Abtrennung in der Regel beispielsweise 5 bis 25 Gew% und bevorzugt 10 bis 20 Gew%.

Um zumindest einen Teil des in dem Sumpfaustrag enthaltenen Monomeren noch vor dem Apparat abzutrennen kann es optional sinnvoll sein, den Rückstand vor Einleiten in den Apparat einer Destillation zu unterwerfen.

Eine derartige, bevorzugt einstufige Destillation erfolgt bevorzugt in einem Fallfilmverdampfer, Kletterverdampfer, Dünnschichtverdampfer, Langrohrverdampfer oder Wendelrohrverdampfer, besonders bevorzugt in einem Fallfilmverdampfer.

Eine derartige einstufige Destillation erfolgt in der Regel bei 80 - 320 °C, bevorzugt 100 - 300 °C und einem Druck von 0,1 - 40 mbar, bevorzugt 0,5 - 20 mbar.

Der diisocyanathaltige Leichtsiederaustrag aus einer solchen einstufigen Destillation kann dann bevorzugt in die Urethanisierung und/oder destillative Aufreinigung geführt werden.

Der dem Apparat zugeführte Rückstand ist in der Regel flüssig, hochviskos oder pastös, bevorzugt flüssig oder hochviskos und weist oft bei einer Temperatur von 150 °C eine Viskosität bis zu 500 mPas gemäß DIN EN ISO 3219 auf.

Erfindungsgemäß wird das Verfahren so ausgeübt, dass der zugeführte Rückstand während seiner gesamten Verweilzeit im Apparat eine hochviskose Flüssigkeit und/oder einen nicht versprödenden Feststoff bildet.

Erfindungsgemäß wird das Verfahren so ausgeübt, dass der den Apparat nach Verarmung an Monomer verlassende Rückstand ohne Abkühlung als eine hochviskose Flüssigkeit, als ein nicht spröder, nicht rieselfähiger, nicht freifließender, kohäsiver, unter Druck teigartig kompaktierender und/oder plastisch deformierbarer Feststoff mit einer Fließgrenze größer 1 N/m², bevorzugt größer 100 N/m² besonders bevorzugt größer 500 N/m² ausgetragen wird.

Die Vorteile des erfindungsgemäßen Verfahrens bestehen darin, daß der Austrag auch ohne die Notwendigkeit des Zusatzes hochsiedender Kohlenwasserstoffe keine Stäube bildet und im erfindungsgemäßen Verfahren auch solche Phasen des Rückstandes aus dem Apparat ausgetragen werden können, die nicht spröde sind, so daß auf eine Abkühlzone innerhalb des Apparates verzichtet werden kann.

Die Dichte dieses Austrags beträgt meist zwischen 500 und 3000 g/l, bevorzugt 600 bis 1000, besonders bevorzugt 700 bis 900 und ganz besonders bevorzugt 750 bis 850 g/l.

Bei den Apparaten, die in dem erfindungsgemäßen Verfahren Anwendung finden, handelt es sich um Apparate mit Zwangsaustrag.

Für eine verbesserte Abtrennung der Monomere bei Durchgang des Rückstandes durch den Apparat ist es bevorzugt, daß dieser eine Bodensteinzahl von mindestens 3, besonders bevorzugt mindestens 5 und ganz besonders bevorzugt mindestens 7 aufweist.

Bevorzugte Ausführungsform derartiger Apparate sind
a) Schaufeltrockner ohne Kühlzone mit Zwangsaustragsorganen,
b) Extruder mit Entgasungsmöglichkeit und
c) vertikale Dünnschichtprozessoren mit Zwangsaustragsorganen.

### a) Schaufeltrockner ohne Kühlzone mit Zwangsaustragsorganen.

Um dem Nachteil des Standes der Technik zu begegnen weisen die erfindungsgemäß einsetzbaren Schaufeltrockner keine Trennung in Heiz- und Kühlzone auf, d.h. eine sprunghafte Absenkung der Temperatur um weit mehr als 100 °C, wie in der US 5962728 beschrieben. Stattdessen steigt die Temperatur des Rückstandes im Verlauf des Durchgangs des Rückstandes durch den Apparat an, bevorzugt um einen Temperaturgradienten, der sich im Verlauf des Durchgangs des Rückstandes durch den Apparat um nicht mehr als 50 °C verändert, und besonders bevorzugt keine wesentliche Temperaturänderung im Verlauf des Durchgangs des Rückstandes durch den Apparat, d.h. weniger als 20 und insbesondere weniger als 10 °C. Dadurch ist der Energiebedarf des Apparates wesentlich gegenüber dem Stand der Technik verringert und es treten keine Anfahrprobleme infolge einer Trennung in eine Heiz- und eine Kühlzone auf.

Derartige Schaufeltrockner sind im wesentlichen horizontal aufgebaut, die Förderung des Rückstandes erfolgt in der Regel über ein- oder zwei Misch- und Knetwellen im Inneren des Apparates. In der Fachliteratur werden diese Apparate auch als Partikelbettreaktor, Knettrockner oder Knetreaktor bezeichnet.

Die Beheizung erfolgt über die Wand und kann in beliebiger Art und Weise erfolgen. Bevorzugt erfolgt die Beheizung nicht nur über die Aussenwandung des Apparates, sondern auch über die Einbauten wie Reinigungshaken, Segmentierscheiben, und Knetwelle.

Die thermische Energie, die über die Wandungen in den Rückstand eingetragen wird, beträgt üblicherweise mehr als 120 kJ/kg Rückstand und weniger als 2400 kJ/kg Rückstand, bevorzugt mehr als 220 kJ/kg Rückstand und weniger als 1800 kJ/kg Rückstand, besonders bevorzugt mehr als 300 kJ/kg Rückstand und weniger als 1400 kJ/kg Rückstand und ganz besonders bevorzugt mehr als 360 kJ/kg Rückstand und weniger als 900 kJ/kg Rückstand.

Die Aufheizstrecke des auf den Schaufeltrockner aufgegebenen Rückstandsstromes beträgt bevorzugt mehr als 10 % und weniger als 70 % der gesamten Länge des Schaufeltrockners, bevorzugt mehr als 20 % und weniger als 60 %, besonders bevorzugt mehr als 30 % und weniger als 50 % der gesamten Länge des Schaufeltrockners.

Derartige Apparate werden beispielsweise von der Firma List AG, Arisdorf, Schweiz, unter den Handelsnamen Discotherm® B oder List-CRP bzw. AP, sowie von der Firma Buss-SMS-Canzler GmbH, Butzbach, Deutschland unter den Namen Reasol® oder Reactotherm® angeboten.

Als Austragsorgane für den Zwangsaustrag des auch nach der Abtrennung des Monomeren erfindungsgemäß hochviskos flüssigen und/oder nicht versprödenden festen monomerabgereicherten Rückstandes können beispielsweise Schnecken, bevorzugt Doppelschnecken dienen.

Des weiteren wird der Schaufeltrockner bevorzugt mit einem Brüdenkondensator betrieben, mit dem das abgetrennte Monomer wiedergewonnen werden kann.

Es stellt eine bevorzugte Ausführungsform des vorliegenden Verfahrens dar, das nutzbare Volumen des Schaufeltrockners nur zu 25 bis 90 %, bevorzugt 30 bis 80%, besonders bevorzugt 40 bis 75% und ganz besonders bevorzugt 50 bis 70% mit Rückstand zu befüllen.

Dies ist insofern vorteilhaft, als daß durch einen derartigen unvollständigen Füllgrad ein Aufschäumen des Rückstandes im Apparat bis zu einem gewissen Grade möglich ist.

### b) Extruder mit Entgasungsmöglichkeiten

Alternativ kann die Abtrennung der Monomeren auch in einem Extruder erfolgen, der mit mindestens einer Entgasungsmöglichkeit, beispielsweise einem Entgasungsdom, versehen ist, über die abgetrenntes Monomer abgeleitet werden kann.

Dazu wird der Rückstand im Vakuum und bei der angegebenen Temperatur gegen beispielsweise eine Loch- oder Schlitzblende gefördert. Durch die Knetung im Inneren des Extruders erfolgt eine Durchmischung des Rückstandes in der Weise, daß das Monomer ausgetrieben wird und über die Entgasungsmöglichkeit aus dem Extruder entfernt wird. Dort wird es dann in an sich bekannter Weise kondensiert und verwertet.

Diese Alternative ist insbesondere dann bevorzugt, wenn sich nur noch kleine Mengen abzutrennendes Monomer in dem Rückstand befinden, beispielsweise 30 Gew% oder weniger.

### c) vertikale Dünnschichtprozessoren mit Zwangsaustragsorganen.

Derartige Dünnschichtprozessoren sind im wesentlichen vertikal ausgerichtet, so daß hier das Erdschwerefeld als Fördergradient für den Rückstand innerhalb des Apparates wirkt, solange der Rückstand unter den Bedingungen innerhalb des Apparates fließfähig ist.

Durch geeignete mechanische Vorrichtungen, beispielsweise Wischblätter, wird der Rückstand auf der beheizten Oberfläche als dünner Produktfilm aufgetragen und verteilt, so daß das leichtflüchtige Monomer abgetrennt werden kann.

Falls erforderlich kann die Abtrennung zusätzlich noch durch Durchleiten eines unter den Abtrennbedingungen inerten Gases unterstützt werden, bevorzugt durch Stickstoff.

Der Brüden kann dabei am Kopf (im Gegenstrom) oder am Fuß (im Gleichstrom) des Dünnschichtprozessors abgenommen werden und wird anschließend in an sich bekannter Weise kondensiert und verwertet.

Mit vertikalen Dünnschichtprozessoren kann der Rückstand beispielsweise bis zu einer Viskosität von bis zu 15.000, bevorzugt bis zu 10.000 Pas gemäß DIN EN ISO 3219 verarbeitet werden.

Die Förderung sollte bevorzugt noch durch scheroptimierte Rotoren unterstützt werden, so daß die Wischblätter (Rotoren) nicht nur auf der beheizten Oberfläche einen dünner Produktfilm erzeugen, sondern den Rückstand auch innerhalb des Apparates zwangsfördern. Dies kann beispielsweise durch in Förderrichtung angeschrägte Wischblätter erfolgen.

Wenn der hochviskos-flüssige und/oder nicht-versprödende feste, monomerabgereicherte Rückstand nach Abtrennung des Monomers am unteren Ende des Dünnschichtprozessors angekommen ist, reicht der natürliche wie auch der zusätzlich Fördergradient in der Regel nicht mehr zur Förderung aus. Dazu wird der Austrag dann am Fuß mittels geeigneter Zwangsaustragssysteme ausgetragen, beispielsweise Schnecken oder Wellen.

Derartige Dünnschichtprozessoren werden beispielsweise von der Firma Buss-SMS-Canzler GmbH, Butzbach, Deutschland unter den Namen Filmtruder® oder Viscon® angeboten.

Diese Alternative ist insbesondere dann bevorzugt, wenn sich noch größere Mengen abzutrennendes Monomer in dem Rückstand befinden, beispielsweise 40 Gew% oder mehr.

Allen Alternativen ist gemein, daß das abgetrennte, Monomer nach Entfernen aus dem Apparat und Kondensation bevorzugt in die Aufreinigung des Diisocyanats eingespeist werden kann. Dazu wird es mit einem Strom aus der Produktion des Diisocyanats oder einem Strom in der Destillativen Reinigung vereinigt, der eine möglichst ähnliche Zusammensetzung aufweist.

Alternativ kann das abgetrennte Monomer bei phosgenfreien Prozessen auch in die Urethanisierung und/oder Carbamatbildung rückgeführt werden, gegebenenfalls nach vorheriger Reurethanisierung oder Recarbamatbildung, d.h. Umsetzung des abgetrennten Monomeren mit Alkohol. Eine weitere Möglichkeit besteht darin, das abgetrennte Monomer in die Carbamat- oder Urethanspaltung zurückzuführen. Daher ist es bei phosgenfreien Prozessen erfindungsgemäß bevorzugt, den gasförmigen, aus dem Apparat abgetrennten Brüden mit einem Alkohol zu quenchen, bevorzugt dem Alkohol, mit dem das Monomer in dem phosgenfreien Prozeß urethanisiert wird. Dazu wird eine ausreichende Menge Alkohol verwendet, um den Brüden in Lösung zu bringen oder zumindest zu suspendieren.

Es kann unabhängig von der Herstellung des Diisocyanats auch sinnvoll sein, den Brüden gasförmig zurückzuführen. Dazu ist es sinnvoll, die Rückführung begleitzubeheizen, um eine unerwünschte Kondensation zu vermeiden. Die Temperaratur wird dabei so gewählt, dass sie kleiner oder gleich der Temperatur des Rückstands im erfindungsgemäßen Apparat ist und größer oder gleich der Siedetemperatur bei dem gewählten Betriebsdruck im Gasraum des zurückzugewinnenden Isocyanats ist.

In einer alternativen, wenn auch weniger bevorzugten Ausführungsform ist es erfindungsgemäß möglich, dem Rückstand vor oder während der Behandlung im Apparat mindestens einen zusätzlichen Stoff zuzugeben, der das Abdestillieren des Monomeren erleichtert und/oder zu einer höheren Viskosität des Austrags aus dem Apparat führt.

Dies können beispielsweise Diphenylether-Biphenyl-Gemische (sog. Diphyl), N-methyl-pyrrolidon, Tetradekalin, hochsiedende Kohlenwasserstoffgemische, insbesondere aromatische Kohlenwasserstoffgemische, beispielsweise Solvesso ® 200 der Firma ExxonMobil Chemical, Kristallöl 60: CAS-Nr. 64742-82-1), schweres Solventnaphtha (Siedebereich etwa 225 - 300 °C) oder Waschöl sein. Bevorzugt wird das Verfahren ohne Zugabe zusätzlicher Stoffe durchgeführt.

Nach Austrag aus dem Apparat kann der monomerabgereicherte Rückstand entsorgt, beispielsweise deponiert oder verbrannt werden. Dabei ist die Konsistenz des monomerabgereicherten Rückstandes außerhalb des Apparates unwesentlich, d.h. eine Erstarrung und/oder ein Verspröden außerhalb des Apparates ist möglich, ohne daß dies erfindungsgemäß von Nachteil ist, solange der Rückstand innerhalb des Apparats nicht versprödet.

## Patentansprüche

1. Verfahren zur Abtrennung von Monomeren aus einem diisocyanathaltigen Rückstand, in mindestens einem Apparat, in dem der Rückstand während der gesamten Verweilzeit im Apparat bei einer Temperatur zwischen 210 und 330 °C und einem Druck unter 300 hPa eine hochviskose Flüssigkeit und/oder einen nicht-versprödenden Feststoff bildet und durch eine Zwangsförderung aus diesem Apparat ausgetragen wird, **dadurch gekennzeichnet, daß** es sich bei dem Diisocyanat um ein (cyclo)aliphatisches handelt.

2. Verfahren gemäß Anspruch 1, **dadurch gekennzeichnet, daß** die Temperatur sich im Verlauf des Durchgangs des Rückstandes durch den Apparat um nicht mehr als 50 °C verändert.

3. Verfahren nach Anspruch 1 oder 2, **dadurch gekennzeichnet, daß** das (cyclo)aliphatische Diisocyanat ausgewählt ist aus der Gruppe 1,6-Diisocyanatohexan, 4,4'- oder 2,4'-Di(isocyanatocyclohexyl)methan und 1-Isocyanato-3,3,5- tri-methyl-5-(isocyanatomethyl)cyclohexan.

4. Verfahren nach einem der vorstehenden Ansprüche, **dadurch gekennzeichnet, daß** man das Diisocyanat durch Phosgenierung von Diaminen erhält.

5. Verfahren nach einem der Ansprüche 1 bis 3, **dadurch gekennzeichnet, daß** man das Diisocyanat in einem phosgenfreien Verfahren herstellt.

6. Verfahren nach Anspruch 5, **dadurch gekennzeichnet, daß** man das Diisocyanat durch Spaltung von Diurethanen erhält.

7. Verfahren nach Anspruch 6, **dadurch gekennzeichnet, daß** man neben monomerem Diisocyanat auch dessen Monourethan, Diurethan, Uretdion, Biuret und/oder Allophanat abtrennt.

8. Verfahren nach einem der vorstehenden Ansprüche, **dadurch gekennzeichnet, daß** der Gehalt an Monomeren im Rückstand bis zu 90 Gew% beträgt.

9. Verfahren nach einem der vorstehenden Ansprüche, **dadurch gekennzeichnet, daß** der Gehalt an Monomeren im monomerabgereicherten Rückstand, der aus dem Apparat gefördert wird, weniger als 20 Gew% beträgt.

10. Verfahren nach einem der vorstehenden Ansprüche, **dadurch gekennzeichnet, daß** der Apparat ausgewählt ist aus der Gruppe bestehend aus
a) Schaufeltrockner ohne Kühlzone mit Zwangsaustragsorganen,
b) Extruder mit Entgasungsmöglichkeiten und
c) vertikale Dünnschichtprozessoren mit Zwangsaustragsorganen.

11. Verfahren nach einem der vorstehenden Ansprüche, **dadurch gekennzeichnet, daß** man den den Apparat nach Verarmung an Monomer verlassenden Rückstand ohne Abkühlung als eine hochviskose Flüssigkeit, als einen nicht spröden, nicht rieselfähigen, nicht freifließenden, kohäsiven, unter Druck teigartig kompaktierenden und/oder plastisch deformierbaren Feststoff mit einer Fließgrenze größer 1 N/m² austrägt.

12. Verfahren nach einem der Ansprüche 1 bis 4 oder 8 bis 11, **dadurch gekennzeichnet, daß** bei Einsatz von Rückständen von Isocyanaten, die einen Gesamtchlorgehalt von 100-700 mg/kg aufweisen, der Apparat zumindest teilweise an den thermisch belasteten Stellen, an denen der Apparat dem chlorhaltigen Rückstand ausgesetzt ist, aus Stahl gefertigt ist, der mindestens 10,5 % Chrom und höchstens 1,2 % Kohlenstoff enthält.

## Claims

1. A process for separating monomers from a diisocyanate-comprising residue in at least one apparatus in which the residue forms a high-viscosity liquid and/or a nonbrittle solid during the total residence time in the apparatus at a temperature of from 210 to 330°C and a pressure below 300 hPa and is discharged from this apparatus by forced transport, wherein the diisocyanate is a (cyclo)aliphatic diisocyanate.

2. The process according to claim 1, wherein the temperature does not change by more than 50°C during passage of the residue through the apparatus.

3. The process according to claim 1 or 2, wherein the (cyclo)aliphatic diisocyanate is selected from the group consisting of 1,6-diisocyanatohexane, 4,4'-or 2,4'-di(isocyanatocyclohexyl)methane and 1-iso-cyanato-3,3,5-trimethyl-5-(isocyanatomethyl)cyclo-hexane.

4. The process according to any of the preceding claims, wherein the diisocyanate is obtained by phosgenation of diamines.

5. The process according to any of claims 1 to 3, wherein the diisocyanate is prepared in a phosgene-free process.

6. The process according to claim 5, wherein the diisocyanate is obtained by dissociation of diurethanes.

7. The process according to claim 6, wherein not only the monomeric diisocyanate but also its monourethane, diurethane, uretdione, biuret and/or allophanate is separated off.

8. The process according to any of the preceding claims, wherein the content of monomers in the residue is up to 90% by weight.

9. The process according to any of the preceding claims, wherein the content of monomers in the residue which has been depleted in monomer and is discharged from the apparatus is less than 20% by weight.

10. The process according to any of the preceding claims, wherein the apparatus is selected from the group consisting of
a) paddle dryers without cooling zone and with forced discharge devices,
b) extruders with venting facilities and
c) vertical thin-film processors with forced discharge devices.

11. The process according to any of the preceding claims, wherein the residue leaving the apparatus after being depleted in monomer is discharged without cooling as a high-viscosity liquid, as a nonbrittle, cohesive solid which does not undergo powder flow and is not free-flowing and under pressure can be compacted in a paste-like fashion and/or be plastically deformed and has a yield point of greater than 1 N/m².

12. The process according to any of claims 1 to 4 or 8 to 11, wherein, when isocyanate residues having a total chlorine content of 100-700 mg/kg are used, the apparatus is at least partly, at the thermally stressed places at which the apparatus is exposed to the chlorine-comprising residue, made of steel comprising at least 10.5% of chromium and not more than 1.2% of carbon.

## Revendications

1. Procédé de séparation de monomères d'un résidu contenant du diisocyanate, dans au moins un appareil, selon lequel le résidu forme pendant la totalité du temps de séjour dans l'appareil à une température comprise entre 210 et 330 °C et une pression inférieure à 300 hPa un liquide hautement visqueux et/ou un solide non fragile, et est déchargé de cet appareil par un transport forcé, **caractérisé en ce que** le diisocyanate est (cyclo)aliphatique.

2. Procédé selon la revendication 1, **caractérisé en ce que** la température ne se modifie pas de plus de 50 °C au cours du passage du résidu dans l'appareil.

3. Procédé selon la revendication 1 ou 2, **caractérisé en ce que** le diisocyanate (cyclo)aliphatique est choisi dans le groupe constitué par le 1,6-diisocyanatohexane, le 4,4'- ou 2,4'-di(isocyanatocyclohexyl)méthane et le 1-isocyanato-3,3,5-triméthyl-5-(isocyanatométhyl)cyclohexane.

4. Procédé selon l'une quelconque des revendications précédentes, **caractérisé en ce que** le diisocyanate est obtenu par phosgénation de diamines.

5. Procédé selon l'une quelconque des revendications 1 à 3, **caractérisé en ce que** le diisocyanate est fabriqué par un procédé sans phosgène.

6. Procédé selon la revendication 5, **caractérisé en ce que** le diisocyanate est obtenu par clivage de diuréthanes.

7. Procédé selon la revendication 6, **caractérisé en ce qu'**en plus du diisocyanate monomère, son monouréthane, son diuréthane, son uretdione, son biuret et/ou son allophanate sont également séparés.

8. Procédé selon l'une quelconque des revendications précédentes, **caractérisé en ce que** la teneur en monomères dans le résidu est de jusqu'à 90 % en poids.

9. Procédé selon l'une quelconque des revendications précédentes, **caractérisé en ce que** la teneur en monomères du résidu appauvri en monomères déchargé de l'appareil est de moins de 20 % en poids.

10. Procédé selon l'une quelconque des revendications précédentes, **caractérisé en ce que** l'appareil est choisi dans le groupe constitué par
a) les séchoirs à pale sans zone de refroidissement à organes de déchargement forcé,
b) les extrudeuses à possibilités de dégazage et
c) les processeurs à couche mince verticaux à organes de déchargement forcé.

11. Procédé selon l'une quelconque des revendications précédentes, **caractérisé en ce que** le résidu quittant l'appareil après l'appauvrissement en monomère est déchargé sans refroidissement sous la forme d'un liquide hautement visqueux, d'un solide non fragile, non ruisselant, non fluide, cohésif, se compactant sous pression en pâte et/ou plastiquement déformable, ayant une limite d'écoulement de plus de 1 N/m².

12. Procédé selon l'une quelconque des revendications 1 à 4 ou 8 à 11, **caractérisé en ce que**, lors de l'utilisation de résidus d'isocyanates qui présentent une teneur totale en chlore de 100 à 700 mg/kg, l'appareil est fabriqué en un acier qui contient au moins 10,5 % de chrome et au plus 1,2 % de carbone au moins en partie aux emplacements sollicités thermiquement, auxquels l'appareil est exposé au résidu contenant du chlore.
